# EUROPÄISCHE PATENTSCHRIFT

(11) **EP 0 548 920 B1**
(45) Veröffentlichungstag und Bekanntmachung des Hinweises auf die Patenterteilung: **22.03.1995**
(21) Anmeldenummer: 92121815.2
(22) Anmeldetag: 22.12.1992
(51) Int. Cl.: B01J 23/84, C07C 29/149

(54) **Verfahren zur Herstellung von höheren Alkoholen durch katalytische Hydrierung von Carbonsäurealkylestern**
Process for the production of high alcohols by catalytic hydrogenation of alkyl esters of carboxylic acids
Procédé de préparation d'alcools superieurs par hydrogenation catalytique d'esters alkyliques d'acides carboxyliques

(30) Priorität: 23.12.1991 DE 4142899
(43) Veröffentlichungstag der Anmeldung: 30.06.1993
(73) Patentinhaber: SÜD-CHEMIE AG, D-80333 München (DE)
(72) Erfinder: Schneider, Michael, Dr., W-8012 Ottobrunn (DE); Kochloefl, Karl, Dr., W-8206 Bruckmühl/Heufeld (DE); Maletz, Gerd, Dr., W-8206 Bruckmühl (DE)
(74) Vertreter: Reitzner, Bruno, Dr.

(56) Entgegenhaltungen:
- EP-A- 0 008 767
- EP-A- 0 034 338
- DD-A- 284 167
- FR-A- 1 408 438
- GB-A- 2 025 252

## Beschreibung

Die Erfindung betrifft ein Verfahren zur Herstellung von höheren Alkoholen durch katalytische Hydrierung von Carbonsäurealkylestern.

Fettalkohole, d.h. aliphatische, vorwiegend lineare, primäre Alkohole mit Kettenlängen von mehr als 8 Kohlenstoffatomen stellen bedeutende Zwischenprodukte der chemischen Industrie dar. Verwendung finden sie bevorzugt zur Herstellung von Tensiden wie z.B. Fettalkoholsulfaten, Polyglykolethern oder Polyglykolethersulfaten.

Wichtiger Rohstoff für ihre Produktion sind Fettsäuren bzw. Fettsäureester, wie sie als Gemische unterschiedlicher Kettenlängen u.a. aus natürlichen Fetten und Ölen erhalten werden können. Die Überführung in die Fettalkohole erfolgt durch katalytische Druckhydrierung.

Die Hydrierungsreaktion wird als Suspensionshydrierung, als Gasphasenhydrierung oder in der Rieselphase durchgeführt. Ausreichend hohe Reaktionsgeschwindigkeiten werden erst bei Drücken oberhalb 250 bar und Temperaturen oberhalb 200°C erreicht. In der Regel werden die Triglyceride vor der Hydrierung nach bekannten Methoden mit Alkoholen, insbesondere mit Methanol umgeestert, die freien Fettsäuren verestert.

Für die Hydrierung von Fettsäuremethylestern zeichnet sich ein Trend zur Reaktionsführung in der Rieselphase ab.

Je nachdem, ob die Ester vor der Hydrierung einer destillativen Reinigung unterzogen werden oder nicht, enthalten sie eine mehr oder weniger hohe Restkonzentration an freien Carbonsäuren. Die Auswahl eines geeigneten Hydrierkatalysators wird nun maßgeblich mitbeeinflußt vom Reinheitsgrad der eingesetzten Ester. In der Technik haben sich gegenwärtig Katalysatoren auf Basis Kupfer-Chrom bzw. Kupfer-Zink bewährt.

Eine wichtige Kenngröße für die Beurteilung der Katalysatorleistungsfähigkeit ist neben der Hydrieraktivität vor allem die Selektivität. Bei Verwendung der bekannten Katalysatoren zur Hydrierung von Fettsäureestern in der Rieselphase kommt es zur Bildung von Methan, höheren Kohlenwasserstoffen und Dialkylethern. Je nach der zum Erreichen einer befriedigend hohen Reaktionsgeschwindigkeit erforderlichen Reaktionstemperatur werden diese Nebenprodukte in relativ hoher Konzentration gebildet und müssen aus dem Reaktionsgemisch durch aufwendige Trennoperationen entfernt werden.

Aus der DE-A-29 28 435 ist ein Verfahren zur Herstellung eines zur Synthese von Methanol geeigneten Kupferkatalysators bekannt, der neben Kupferoxid auch Zinkoxid, Aluminium- oder Chromoxid und Manganoxid enthält. Die Verwendung von Chromoxid ist jedoch aus Gründen des Umweltschutzes bedenklich. Aluminiumoxid wird bei Katalysatoren für die Herstellung von höheren Alkoholen nicht benötigt. Außerdem wird die Methanolsynthese gewöhnlich bei Drücken von nicht mehr als 20 bar durchgeführt.

Aus der DD-A-284 167 ist ein Verfahren zur Herstellung von Katalysatoren für die Synthese von C₂- bis C₅-Alkoholen bekannt, wobei Katalysatoren auf der Basis von Oxiden des Kupfers, Zinks, Mangans und/oder Aluminiums verwendet werden. Die bevorzugten Katalysatoren haben einen Kupferanteil von etwa 70 %.

Aufgabe der vorliegenden Erfindung war es, die genannten Nachteile der bekannten Katalysatoren zu vermeiden und ein Verfahren zur Herstellung von Alkoholen durch katalytische Hydrierung von Carbonsäurealkylestern zur Verfügung zu stellen, das sich durch eine deutlich erhöhte Selektivität auszeichnet.

Diese Aufgabe konnte überraschenderweise gelöst werden durch ein Verfahren, das dadurch gekennzeichnet ist, daß man einen Katalysator verwendet, dessen oxidische Vorstufe die Zusammensetzung

CuZn_{0,5-2}Mn_{0,01-0,5}Oₓ

hat, wobei x die zur Sicherstellung der Elektroneutralität erforderlichen Sauerstoffatome pro Formeleinheit bezeichnet; und daß man die Umsetzung bei einer Temperatur von 150 bis 250°C und einem Druck von 100 bis 400 bar bis zu einer Verseifungszahl von maximal 5 durchführt.

Vorzugsweise führt man die Umsetzung bei einer Temperatur von 180 bis 230°C und einem Druck von 200 bis 350 bar durch.

Vorzugsweise hat der Katalysator die Zusammensetzung

CuZn_{0,8-1,5}Mn_{0,05-0,2}Oₓ.

Vorzugsweise werden Katalysator-Formkörper verwendet, deren spezifisches Porenvolumen (in der oxidischen Vorstufe) 0,15 bis 1,2, vorzugsweise 0,20 bis 0,70 cm³/g beträgt.

Das spezifische Porenvolumen wird nach der Methode der Quecksilberpenetration bestimmt, die bei J. van Brakel et al., Powder Technology 29 (1981),1 beschrieben ist. Bei dieser Methode wird das Quecksilber bis zu einem Druck von etwa 2000 bar in die Katalysator-Formkörper eingepreßt, wobei die Volumenabnahme des Quecksilbers als Funktion des Druckes aufgetragen wird. Auf diese Weise erhält man eine Kurve, aus deren Verlauf sich auch die Porenverteilung ermitteln läßt. Nach der Quecksilberpenetrationsmethode kann nur das Volumen und die Verteilung der Poren mit einem Durchmesser von > 7,5 nm bestimmt werden.

Der verwendete Katalysator hat (in der oxidischen Vorstufe) vorzugsweise eine BET-Oberfläche von 20 bis 180 m²/g, insbesondere von 40 bis 120 m²/g. Die BET-Oberfläche wird über die N₂-Sorption nach der Einpunkt-Methode bestimmt, wie es in DIN 66 132 angegeben ist.

Die mittlere Seitendruckfestigkeit des verwendeten Katalysators beträgt normalerweise in der oxidischen Form mindestens 40 N, insbesondere mindestens 60 N. Die Seitendruckfestigkeit wird an einem zylindrischen Formkörper mit einem Durchmesser von 4,5 mm und einer Länge von 4,5 mm unter Verwendung des Tablettenprüfgeräts 4 M der Firma Schleuniger bestimmt. Der zylindrische Formkörper wird zwischen die Backen der Vorrichtung gelegt, wobei ein Druck senkrecht zur Zylinderachse ausgeübt wird. Die Kraftsteigerungsrate beträgt 20 N/sec.

Die Katalysator-Formkörper werden z.B. als Zylinder, Kugeln, Ringe, Wagenräder, polylobäre oder Wabenkörper verwendet.

Die Herstellung des erfindungsgemäß verwendeten Katalysators kann grundsätzlich nach bekannten Methoden erfolgen, vorausgesetzt, es wird eine ausreichend hohe Wechselwirkung der Katalysatorkomponenten erreicht.

Bevorzugte Herstellmethoden sind z.B. Mischen bzw. Naßvermahlen der Kupfer-, Zink- und Manganoxide, gefolgt von der Verformung und gegebenenfalls einer thermischen Behandlung. Besonders bevorzugt ist die Fällung geeigneter Zwischenstufen aus Lösungen von Salzen der genannten Metalle. Geeignete Zwischenstufen sind Verbindungen, die sich durch thermische Behandlung in die Oxide überführen lassen, also z.B. die Hydroxide und Carbonate bzw. Hydroxocarbonate. Besonders geeignete Fällungsmittel sind die Alkalicarbonate und -hydrogencarbonate. Als Rohstoffe eignen sich grundsätzlich alle löslichen Salze, wie z.B. die Halogenide, die Sulfate oder Nitrate.

Der Katalysator wird in der reduzierten Form verwendet, wobei die Reduktion in an sich bekannter Weise erfolgt, z.B. in einem wasserstoffhaltigen Gas bei Temperaturen von 100 bis 500°C, vorzugsweise von 150 bis 300°C. Es können auch die Zwischenstufen direkt reduziert werden, ohne daß sie vorher in die Oxide umgewandelt werden. Ferner kann die Reduktion in situ im Hydrierreaktor erfolgen.

Das folgende Beispiel erläutert die Herstellung eines erfindungsgemäßen Katalysators:

### Beispiel 1

1346 g Cu(NO₃)₂*3H₂O, 1826 g Zn(NO₃)₂*6H₂O und 120 g Mn(NO₃)₂*4H₂O werden auf 9 Liter Gesamtvolumen in entsalztem H₂O gelöst und auf 60 °C erhitzt. Diese Nitratlösung pumpt man während 120 min in einen gerührten Fällbehälter; gleichzeitig wird eine 15%ige Sodalösung so dosiert, daß sich im Fällbehälter ein pH-Wert zwischen 6.8 und 7.0 einstellt. Die Temperatur hält man bei 60 °C. Man filtriert und wäscht den Niederschlag durch mehrfaches Resuspendieren bei 40 °C. Bei 120°C trocknet man über Nacht, und calciniert 3 h bei 200 °C, erhöht dann die Temperatur auf 400 °C und beläßt für weitere 3 h bei dieser Temperatur. Das calcinierte Produkt wird gemahlen und die Siebfraktion kleiner 1 mm im Format 4.5 x 4.5 mm tablettiert.

Als Vergleich dient ein kommerzieller Katalysator auf Basis Kupfer-Chrom (Handelsprodukt G-99B der Anmelderin; Metallgehalte Cu 36,5%; Cr 32%; Ba 2,2%; Mn 2,4%).

Die Leistungsfähigkeit der beiden Katalysatoren bei der Hydrierung von Fettsäuremethylestern in der Rieselphase wurde wie folgt bestimmt:
200 ml des Katalysators nach Beispiel 1 werden in einem Rohrreaktor zunächst reduziert: Als Reduktionsgas dient eine Mischung von Wasserstoff und Stickstoff. Der H₂-Gehalt der Mischung beträgt 2%. Die Temperatur wird von 150°C langsam auf 200°C erhöht.

Als Substrat wird ein kommerziell erhältliches Gemisch von Fettsäuremethylestern im Kettenlängenbereich C₁₂ bis C₁₈ verwendet. Die Hydrierung wird bei 300 bar mit einer LHSV (liquid hourly space velocity) von 1 Liter Estergemisch/ Liter Katalysator und Stunde durchgeführt. Als Maß für den Umsatz wird die Abnahme der Verseifungszahl nach DIN 51 559 bestimmt.

Die Hydrierungsaktivität wird durch die Reaktionstemperatur charakterisiert, bei der der gewünschte Umsatz an Fettsäuremethylestern erreicht wird. Die Selektivität wird durch gaschromatographische Analyse der Nebenprodukte bestimmt. Man erhält die folgenden Ergebnisse:

| 1. Umsatz: | | |
|---|---|---|
| | Beispiel 1 | Beispiel A (Vergl.) |
| Verseifungszahl 2 wird erreicht bei | 220°C | 230°C |

| 2. Nebenprodukte bei 230°C Reaktionstemperatur in Mol pro Mol Fettalkohol: | | |
|---|---|---|
| - | Beispiel 1 | Beispiel A (Vergl.) |
| - CH₄ | 2.5 | 5 |
| - höhere Kohlenwasserstoffe | 1 | 2 |
| - Dimethylether | 0 | 3 |

Der erfindungsgemäß verwendeten Katalysator ist also offensichtlich sowohl bezüglich der Hydrieraktivität als auch hinsichtlich der Selektivität deutlich überlegen. Besonders hervorzuheben ist, daß die Dimethyletherbildung vollständig unterdrückt wird.

## Patentansprüche

1. Verfahren zur Herstellung von höheren Alkoholen durch katalytische Hydrierung von Carbonsäurealkylestern, dadurch gekennzeichnet, daß man einen Katalysator verwendet, dessen oxidische Vorstufe die Zusammensetzung
CuZn_{0,5-2}Mn_{0,01-0,5}Oₓ
hat, wobei x die zur Sicherstellung der Elektroneutralität erforderlichen Sauerstoffatome pro Formeleinheit bezeichnet; und daß man die Umsetzung bei einer Temperatur von 150 bis 250°C und einem Druck von 100 bis 400 bar bis zu einer Verseifungszahl von maximal 5 durchführt.

2. Verfahren nach Anspruch 1, dadurch gekennzeichnet, daß man die Umsetzung bei einer Temperatur von 180 bis 230°C und einem Druck von 200 bis 350 bar durchführt.

3. Verfahren nach Anspruch 1 oder 2, dadurch gekennzeichnet, daß man einen Katalysator der Zusammensetzung
CuZn_{0,8-1,5}Mn_{0,05-0,2}Oₓ
verwendet.

4. Verfahren nach einem der Ansprüche 1 bis 3, dadurch gekennzeichent, daß Katalysator-Formkörper verwendet werden, deren Porenvolumen (mittels Hg-Porosimetrie bestimmt) 0,15 bis 1,2, vorzugsweise 0,20 bis 0,70 cm³/g beträgt.

5. Verfahren nach einem der Ansprüche 1 bis 4, dadurch gekennzeichnet, daß man einen Katalysator verwendet, dessen BET-Oberfläche 20 bis 180 m²/g, vorzugsweise 40 bis 120 m²/g beträgt.

6. Verfahren nach einem der Ansprüche 1 bis 5, dadurch gekennzeichent, daß man Katalysator-Formkörper verwendet, deren mittlere Seitendruckfestigkeit in der oxidischen Form mindestens 40 N, vorzugsweise mindestens 60 N beträgt.

7. Verfahren nach einem der Ansprüche 1 bis 6, dadurch gekennzeichnet, daß als Katalysator-Formkörper Zylinder, Kugeln, Ringe, Wagenräder, polylobäre oder Wabenkörper verwendet werden.

## Claims

1. A method for the preparation of higher alcohols by catalytic hydrogenation of carboxylic acid alkyl esters, characterized in that a catalyst is used whose oxidic precursor has the composition
CuZn_{0.5-2}Mn_{0.01-0.5}Oₓ
wherein x represents the oxygen atoms per formula unit necessary to assure electroneutrality, and in that the reaction is carried out at a temperature of 150 to 250°C and at a pressure of 100 to 400 bar up to a saponification number of a maximum of 5.

2. A method according to Claim 1, characterized in that the reaction is carried out at a temperature of 180 to 230°C and at a pressure of 200 to 350 bar.

3. A method according to Claim 1 or 2, characterized in that a catalyst is used having the composition
CuZn_{0.8-1.5}Mn_{0.05-0.2}O_{x.}

4. A method according to any one of Claims 1 to 3, characterized in that moulded catalyst bodies are used whose pore volume (determined by Hg porosimetry) is 0.15 to 1.2, preferably 0.20 to 0.7 cm³/g.

5. A method according to any one of Claims 1 to 4, characterized in that a catalyst is used whose BET surface area is 20 to 180 m²/g, preferably 40 to 120 m²/g.

6. A method according to any one of Claims 1 to 5, characterized in that moulded catalyst bodies are used whose average lateral compression strength in the oxidic form is at least 40 N, preferably at least 60 N.

7. A method according to any one of Claims 1 to 6, characterized in that cylinders, spheres, rings, cartwheels, polylobar or honeycomb-shaped bodies are used as moulded catalyst bodies.

## Revendications

1. Procédé pour préparer des alcools supérieurs par hydrogénation catalytique d'esters alkyliques d'acides carboxyliques, caractérisé en ce qu'on utilise un catalyseur dont le précurseur par oxydation a la composition suivante :
CuZn_{0,5-2}Mn_{0,01-0,5}Oₓ
où x désigne le nombre d'atomes d'oxygène nécessaires, par unité de formule, pour garantir la neutralité électronique, et en ce qu'on met en oeuvre la réaction à une température de 150 à 250°C et sous une pression de 100 à 400 bars jusqu'à un indice de saponification au plus égal à 5.

2. Procédé selon la revendication 1, caractérisé en ce qu'on met en oeuvre la réaction à une température de 180 à 230°C sous une pression de 200 à 350 bars.

3. Procédé selon la revendication 1 ou 2, caractérisé en ce qu'on utilise un catalyseur ayant la composition suivante :
CuZn_{0,8-1,5}Mn_{0,05-0,2}Oₓ

4. Procédé selon l'une des revendications 1 à 3, caractérisé en ce qu'on utilise un catalyseur moulé dont le volume des pores (déterminé par porosimétrie au mercure) est de 0,15 à 1,2 et de préférence de 0,20 à 0,70 cm³/g.

5. Procédé selon l'une des revendications 1 à 4, caractérisé en ce qu'on utilise un catalyseur dont l'aire BET est de 20 à 180 et de préférence de 40 à 120 m²/g.

6. Procédé selon l'une des revendications 1 à 5, caractérisé en ce qu'on utilise un catalyseur moulé dont la résistance moyenne à une compression latérale sous forme oxydée est d'au moins 40 N et de préférence d'au moins 60 N.

7. Procédé selon l'une des revendications 1 à 6, caractérisé en ce qu'on utilise comme catalyseur moulé des cylindres, des sphères, des anneaux, des roues de voitures ou encore des corps polylobaires ou en nids d'abeilles.
